# EUROPEAN PATENT SPECIFICATION

(11) **EP 0 884 021 B1**
(45) Date of publication and mention of the grant of the patent: **15.09.2004**
(21) Application number: 98110684.2
(22) Date of filing: 10.06.1998
(51) Int. Cl.: A61B 5/0416, A61B 5/0408

(54) **Biomedical electrode provided with a press stud**
Biomedizinische Druckknopf-Elektrode
Electrode biomédicale à bouton-pression

(30) Priority: 11.06.1997 JP 15376697
(43) Date of publication of application: 16.12.1998
(73) Proprietor: Omron Healthcare Co., Ltd., Kyoto 615-0084 (JP)
(72) Inventor: Sasaki, Hiroaki c/o Nitto Denko Corp., Osaka (JP); Nishida, Satoshi, c/o Omron Healthcare Co., Ltd., Kyoto 61500-0084 (JP)
(74) Representative: Wilhelms, Rolf E., Dr.

(56) References cited:
- EP-A- 0 627 193
- US-A- 4 706 680
- US-A- 4 934 383
- US-A- 5 402 780

## Description

### 1. Field of the Invention

This invention relates to an electrode employed in a low frequency care device for a living body, and more particularly to an improved electrode provided with a snap capable of preventing generation of any conduction failure.

### 2. Description of the Prior Art

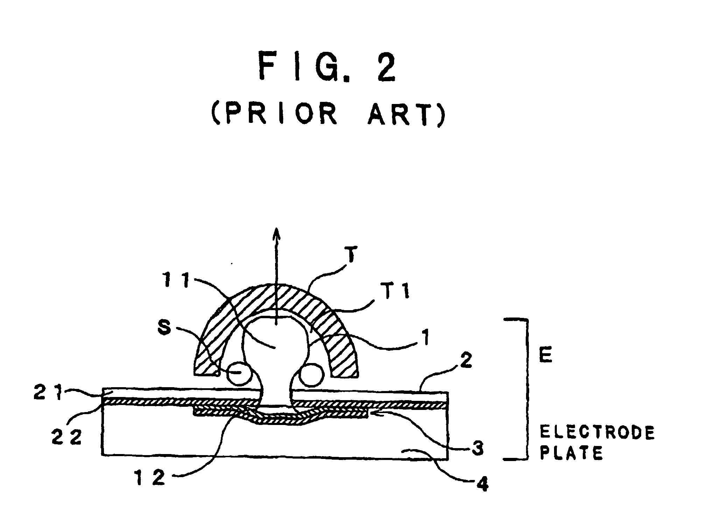

Patent publication JP 8-196644 discloses a conventional electrode pad for a living body employed by a low frequency care device as shown in Fig. 2. The electrode pad includes a snap 1 having a convex portion 11 and a flange 12, a support member 2 having a laminating configuration composed of an insulating layer 21 and an electrically conductive layer 22 which are pierced by the convex portion 11 from the side of the electrically conductive layer 22 to allow the flange 12 to face the conductive layer 22, and an insulating adhesive tape 3 to coat the flange 12 to be fixedly secured to the conduction layer 22, providing an electrode plate E. The electrode pad further includes an electrical conductive hydrated adhesive layer 4 of hydrated gel of acrylic series or urethane series which is disposed on the conductive layer 22 of the electrode plate E.

The electrode pad is connected by a code terminal T to be connected with a low frequency care device. The code terminal T includes a concave portion T1 corresponding to the convex portion 11 of the snap 1, and the snap 1 is connected with the conductive layer 22 by engaging the concave portion T1 with the convex portion 11 and lifting the snap by a spring S to make conduction between the electrode pad and the code terminal T.

The conventional electrode pad, however, is found to have such a problem that the electrode pad invites conduction failure. As a result of investigation about reason for such a problem, it is found that the snap 1 is disabled to be lifted up as the spring S of the code terminal T is repeatedly used because it is widened by the repeated use, thereby the connection between the snap 1 and the conductive layer 22 of the support member 2 being unstable.

### SUMMARY OF THE INVENTION

It is, therefore, a primary object of this invention to provide an improved electrode provided with a snap for a living body, in which the snap for connection with a connector of an external device pierces a support member having a laminating construction composed of an insulating layer and a conductive layer, and an adhesive tape covering a bottom portion of the snap on a side of the conductive layer is electrically conductive.

In the electrode provided with the snap for a living body according to this invention, the adhesive tape covering the bottom of the snap is electrically conductive to make electrical conduction between the snap and the support member, thereby any conduction failure being completely avoided.

### BRIEF DESCRIPTION OF THE DRAWINGS

Other objectives and advantages of this invention will be more readily apparent from the following detailed description provided in conjunction with the following figures, of which:
Fig. 1 is a sectional view of an electrode provided with a snap for a living body as a preferred embodiment of this invention; and
Fig. 2 is a sectional view of a conventional electrode provided with a snap for a living body;

### DESCRIPTION OF THE PREFERRED EMBODIMENT

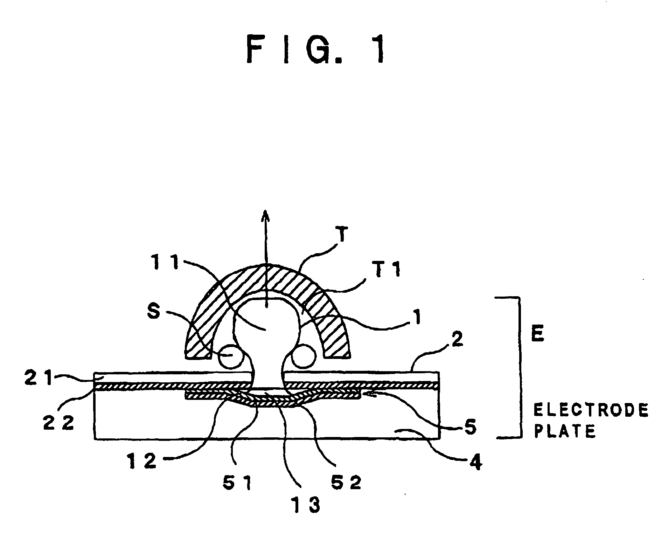

Returning, now, to Fig. 1, there is shown a sectional view of an electrode provided with a snap 1 for a living body as a preferred embodiment of this invention, the snap 1 being adapted to be connected with a connector of an external device such as a low frequency care device.

The snap 1 may employ a nickel plating on brass, a reducing work of stainless steel, or an electrically conductive resin molding product, but is necessary to have corrosion resistance for contacting hydrated adhesive material and durability for repeatedly putting on and taking off. Therefore, a reducing work of stainless steel comprising 8 weight percent nickel is the most suitable to the snap 1.

The support member 2 pierced by snap 1 includes a laminating layer comprised of an insulating layer 21 and an electrically conductive layer 22. The insulating layer 21 of the support member 2 may exemplarily employ various kinds of resin films such as polyolefin resin such as ethylene, polypropylene and so forth, polyvinyl chloride resin, polyester resin and so forth, foam, nonwoven fabric, cloth or a laminate of these. The thickness of the layer 21 is desirable to be 40 to 200 µm in case of a film, and 0.2 to 1 mm in case of a foam member though it depends on the material.

The conductive layer 22 of the support member 2 may employ conductive coating material, metallic foil, metal evaporation, or carbon fiber cloth, in which an appropriate binder contains metal powder such as silver, copper, nickel, or tin, electrically conductive carbon black powder, or Ag/AgCl(silver/silver chloride) powder. In view of the fact that the electrically conductive layer 22 contacts electrically conductive hydrated adhesive material, the layer 22 is desirable to employ electrically conductive carbon black, electrically conductive coating material of Ag/AgCl (silver/silver chloride) or carbon fiber cloth because of their corrosion resistance. The thickness is desirable to be around 0.1 to 30 µm to make a good membrane though it depends on its material.

The flange 12 of the snap 1 is coated by an adhesive tape 5 composed of an electrically conductive adhesive layer 51 and an insulating layer 52 to bring the adhesive layer 51 into contact with a bottom 13 of the snap 1. The insulating layer 52 may employ material similar to that of the insulating layer 21 of the support member 2, but is desirable to employ various kinds of resin films in view of its intensity and thickness. The electrically conductive adhesive layer 51 for connecting the bottom 13 of the snap 1 with the electrically conductive layer 22 of the layer 2 may employ an adhesive composition thing blended by metal powder or electrically conductive carbon black or same composition thing as the electrically conductive hydrated adhesive layer 4 such as hydrated gel of acrylic series and urethane series. The adhesive composition thing blended by metal powder and electrically conductive carbon black cannot obtain sufficient adhesiveness to connect the bottom 13 of the snap 1 with the electrically conductive layer 22 of the support member 2, so that same composition thing as the electrically conductive hydrated adhesive layer 4 is desirable to be employed. The thickness is desirable to be around 10 to 60 µm. If anchoring between the composition thing same as the electrically conductive hydrated adhesive layer 4 and the insulating layer 52 is unsatisfactory, primer coating by ethylene resin imine series polymer or carbon black coating is recommended to be applied.

The electrically conductive adhesive tape 5 has a size larger than the bottom 13 of the snap 1 and smaller than the electrically conductive layer 22 allowing the size to be sufficient contact between the conductive adhesive tape 5 and the conductive layer 22.

The electrode with the snap for the living body shown in Fig. 1 includes an electrode plate E having the snap 1, the support member 2 and the electrically conductive adhesive tape 5, and the electrically conductive hydrated adhesive layer 4 is disposed on the side of the conductive layer 22 of the electrode plate E.

The electrically conductive hydrated adhesive layer 4 is desirable to exemplarily employ karaya gum, gelatine, polyacrylic acid or its salt, other water-soluble acrylic series polymer, polyacrylamide, polyvinyl alcohol, carboxymethyl cellulose, or water-soluble polymer such as polyurethane which is blended by multiple value ethyl alcohol such as glycerine, water or electrolyte and further applied by a bridge forming means. In view of quality stability, adhesiveness, conductivity and configuration holding, the best composition thing is a hydrated gel, in which polyacrylic acid or its salt, e.g. triethanolamine salt is blended by glycerine, water or electrolyte to be applied by a proper bridge forming means. The moisture content of the electrically conductive hydrated adhesive layer 4 is normally 5 to 50 weight percent, preferably 10 to 30 weight percent.

In view of adhesiveness and durability, the thickness of the conductive hydrated adhesive layer 4 is desirable to be 0.1 to 3.0 mm, preferably 0.5 to 2.0 mm. Around center of the adhesive layer 4 in its thickness direction there may be employed nonwoven fabric of thin polyester, polypropylene, rayon, pulp and so forth.

On the electrode plate E side and the opposite side of the adhesive layer 4, there is disposed separate paper which is plastic film or paper on normally one face or both faces thereof applied by silicone resin or fluorocarbon resin, further disposed by desquamation. The living body electrode with a snap of Fig. 1 is connected with a code terminal T representing a connector for an external device such as a low frequency care device. The code terminal T includes a concave T1 corresponding to a convex 11 of the snap 1 of the electrode pad. The concave T1 is engaged with the convex 11 and the snap 1 is lifted by a spring S to make contact between the sap 1 and the conductive layer 22 of the support member 2. Even if the snap cannot be lifted up by the spring S of the code terminal T which is widened by repetition use, there remains an electrical contact among the conductive layer 22 of the support member 2, the snap 1 and the code terminal T.

Thus, in the electrode provided with the snap for the living body according to this invention, even if the snap cannot be lifted up by the spring S of the code terminal connected with the low frequency care product or the like, the adhesive tape is electrically conductive, retaining conduction among the conductive layer of the support member, the snap and the code terminal to completely avoid any conduction failure.

This invention will be described more in detail in conjunction with the embodiment, comparison and experimental data, but the scope of this invention shall not be limited thereby.

The electrode with the snap for the living body of Fig. 1 is made by the following manner; An electrically conductive carbon paint film of thickness 10 µm is employed by the electrically conductive layer 22, a polyester film of thickness 75µm is glued therewith as the insulating film 21, and the support member having a thickness 85 µm is produced. The snap 1 having a height 2.4 mm and a flange diameter 6 mm is produced by reducing work of stainless steel plate having a thickness 0.25 mm of SUS316 (nickel 10 to 40 percent). The support member 2 is further provided with a through hole through which the snap 1 is inserted from the conductive layer side 22 of the support member 2.

Then, the electrically conductive adhesive tape 5 is produced by painting the insulating layer 52 composed of a polyester film of thickness 25 µm with the conductive adhesive layer 51 composed of acrylic series hydrated gel having a thickness 40 µm, and bored a hole in a 10 mm diameter to coat and fix the support member 2 allowing the conductive layer 51 to come into contact with the bottom 13 of the snap 1 to provide the electrode plate E.

The conductive hydrated adhesive layer 4 composed of acrylic series hydrated gel is adjusted to be 1 mm, disposed on the side of the conductive layer 22 of the electrode plate E, and coated by polyester separate paper to be bored a hole of 40 mm square to provide the electrode pad.

For comparison, the conductive adhesive tape 5 of this embodiment is replaced by an insulating adhesive tape having electrically non-conductive ordinary acrylic series adhesive material (thickness 25 µm), but other components are not changed, producing another electrode pad for comparison.

There is further produced an electrode pad in which the portion of the snap 1 contacting the conductive layer 22 of the support member 2 of the electrode pad is coated by electrically insulating paint.

As a result of conductive test by employing a low frequency care device about these electrode pads, the electrode pad for comparison is conductive when no-process is applied but non-conductive when the insulating process is applied. The electrode pad of this embodiment is conductive whether the insulating process is applied or whether no process is applied. Accordingly, the electrode with a snap for the living body of this invention has been confirmed that it is completely avoided from any conductive failure.

## Claims

1. An electrode provided with a snap for a living body comprising
a snap (1) for connection with a connector of an external device,
a support member (2) including a laminating construction comprised of an insulating layer (21) and a conductive layer (22), said snap (1) inserting through the support member (2) from a side of the insulating layer (21),
said electrode being **characterised in that**
a conductive adhesive tape (5) covers a bottom (13) of said snap (1) on a side of said conductive layer (22).

2. An electrode according to claim 1 in which a conductive hydrated adhesive layer (4) is laminated on said conductive adhesive tape (5) and located on a side of the bottom (13) of the snap (1).

3. An electrode according to claim 1 in which said conductive adhesive tape (5) is composed of a conductive adhesive layer (51) and an insulating layer (52).

4. An electrode according to claim 2 in which said conductive adhesive layer (51) of the conductive adhesive tape (5) is a hydrated adhesive material.

5. An electrode according to claim 1 in which said conductive adhesive tape (5) is larger than the bottom (13) of said snap (1) and comes in contact with the conductive layer (22) of said support member (2).

6. Electrode according to claim 1 in which said snap (1) has corrosion resistance.

## Patentansprüche

1. Mit einem Druckknopf versehene Elektrode für einen lebenden Körper, welche aufweist:
einen Druckknopf (1) mit einem Verbindungsstück einer externen Vorrichtung,
einem Trägerelement (2), welches einen Schichtaufbau aus einer Isolationsschicht (21) und einer leitenden Schicht (22) enthält, wobei sich der Druckknopf (1) durch das Trägerelement (2) hindurch von einer Seite der Isolationsschicht (21) her einführt,
wobei die Elektrode **dadurch gekennzeichnet ist, dass**
ein leitfähiges Klebeband (5) einen Boden (13) des Druckknopfes (1) auf einer Seite der leitfähigen Schicht (22) abdeckt.

2. Elektrode nach Anspruch 1, bei welcher eine leitfähige hydrierte Klebeschicht (4) auf das leitfähiges Klebeband (5) aufgeschichtet und auf einer Seite des Bodens (13) des Druckknopfs (1) angeordnet ist.

3. Elektrode nach Anspruch 1, bei welcher das leitfähige Klebeband (5) aus einer leitfähigen Klebeschicht (51) und einer Isolationsschicht (52) zusammengesetzt ist.

4. Elektrode nach Anspruch 2, bei welcher die leitfähige Klebeschicht (51) des leitfähigen Klebebands (5) ein hydriertes Klebematerial ist.

5. Elektrode nach Anspruch 1, bei welcher das leitfähige Klebeband (5) größer als der Boden (13) des Druckknopfs (1) ist und mit der leitfähigen Schicht (22) des Trägerelements (2) in Berührung kommt.

6. Elektrode nach Anspruch 1, bei welcher der Druckknopf (1) Korrosionsfestigkeit hat.

## Revendications

1. Electrode pourvue d'un bouton-pression pour un corps vivant comprenant :
un bouton-pression (1) pour une liaison à un connecteur d'un dispositif externe,
un élément de support (2) comprenant une construction stratifiée comprenant une couche isolante (21) et une couche conductrice (22), ledit bouton-pression (1) s'insérant à travers l'élément de support (2) à partir d'un côté de la couche isolante (21),
ladite électrode étant **caractérisée en ce que** :
une bande adhésive conductrice (5) recouvre une partie de fond (13) dudit bouton-pression d'un côté de ladite couche conductrice (22).

2. Electrode selon la revendication 1, dans laquelle une couche adhésive conductrice hydratée (4) est lamifiée sur ladite bande adhésive conductrice (5) et située d'un côté de la partie de fond (13) du bouton-pression (1).

3. Electrode selon la revendication 1, dans laquelle ladite bande adhésive conductrice (5) est composée d'une couche adhésive conductrice (51) et d'une couche isolante (52).

4. Electrode selon la revendication 2, dans laquelle ladite couche adhésive conductrice (51) de la bande adhésive conductrice (5) est un matériau adhésif hydraté.

5. Electrode selon la revendication 1, dans laquelle ladite bande adhésive conductrice (5) est plus grande que la partie de fond (13) dudit bouton-pression (1) et vient en contact avec la couche conductrice (22) dudit élément de support (2).

6. Electrode selon la revendication 1, dans laquelle ledit bouton-pression (1) est résistant à la corrosion.
